(19) Europäisches Patentamt
European Patent Office
Office européen des brevets

(11) Veröffentlichungsnummer: **0 087 393**
**B1**

(12) **EUROPÄISCHE PATENTSCHRIFT**

(45) Veröffentlichungstag der Patentschrift: **14.01.87**

(51) Int. Cl.⁴: **C 07 D 251/54,** C 08 K 5/34

(21) Anmeldenummer: **83810068.3**

(22) Anmeldetag: **17.02.83**

(54) **Verfahren zur Herstellung von Isomelaminen und deren Verwendung.**

(30) Priorität: 23.02.82 CH 1105/82

(43) Veröffentlichungstag der Anmeldung: **31.08.83 Patentblatt 83/35**

(45) Bekanntmachung des Hinweises auf die Patenterteilung: **14.01.87 Patentblatt 87/3**

(84) Benannte Vertragsstaaten: **CH DE FR GB LI**

(56) Entgegenhaltungen:
**US-A-2 485 983**

**JOURNAL OF THE CHEMICAL SOCIETY, Sektion C, 1971, Seiten 3827-3829, London, GB, G. RAPI u.a.: "Dimethylcarbodi-imide. Improved synthesis, cyclic trimer, and reaction with alpha-hydroxy-esters"**

(73) Patentinhaber: **CIBA- GEIGY AG, Klybeckstrasse 141, CH- 4002 Basel (CH)**

(72) Erfinder: **Lohse, Friedrich, Prof. Dr., Buchenstrasse 23, CH- 4104 Oberwil (CH)**
Erfinder: **Zondler, Helmut, Dr., Oberwilerstrasse 49, CH- 4103 Bottmingen (CH)**

**EP 0 087 393 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Vorliegende Erfindung vetrifft ein Verfahren zur Herstellung von Isomelaminen und ein Verfahren zum Härfen von Epoxidharzen unter Verwendung dieser Verbindungen.

In der DE-PS 1 951 650 wird zur Verbesserung der Anfärbbarkeit von Fäden, Fasern und Garnen aus Polyestern vorgeschlagen, mit Isomelaminen modifizierte Polyester zu verwenden. Isomelamine sind bisher nur durch mehrstufige und aufwendige Synthesen mit zum Teil geringen Ausbeuten erhältlich gewesen. Beispielsweise werden Synthesen für Iscmelamine in J. appl. Chem., 6, March, 19;6, S9-93; J. Org. Chem., 25, (1960), 1043-10;5 und J. Chem. Soc. (C), 1971, 3827-3829 beschrieben.

Es wurde nun gefunden, dass sich Isomelarine in einfacherer und wirtschaftlicherer Weise herstellen lassen, indem man N-Cyano-N-alkylcarbonsäureamide, welche stabile Verbindungen darsteilen, unter bestimmten Reaktionsbedingungen zu Isomelaminen umsetzt.

Gegenstand der vorliegenden Erfindung ist somit ein Verfahren zur Herstellung von Isomelaminen der Formel I

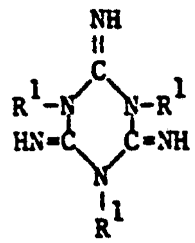

(I),

worin jedes $R^1$ fur éin Alkyl mit 1 bis 8 C-Atomen, Arakyl mit bis zu 12 C-Atomen, Allyl oder Methallyl steht, dadurch gekennzeichnet, dass man N-Cyano-N-(ar)alkyl- oder N-Cyano-N-(meth)allylcarbonsäureamide der Formel II oder III

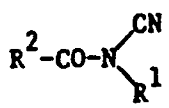

(II) oder

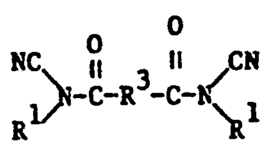

(III),

worin jedes R die gleiche Bedeutung wie in Formel 1 hat, R ein Wasserstoffatom, Alkyl mit 1 bis 16 C-Atomen, Cycloalkyl mit 5 bis 8 C-Atomen oder Aryl mit 6 oder 10 Ring-C-Atomen bedeutet und R für eine direkte Bindung oder ein Alkylen mit 1 bis 12 C-Atomen steht, mit

a) wässriger Natron-, Kalilauge oder Ammoniaklösung oder

b) primären aliphatischen oder cycloaliphatischen Aminen in einem Lösungsmittel oder mit

c) primären aliphatischen Alkoholen in Gegenwart von katalytischen Mengen einer basischen Verbindung

im Temperaturbereich von -20°C bis 200°C umsetzt, wobei man auf 3 Carbonsäureamidgruppenäquivalente mindestens 3 Hydroxyl- oder Aminogruppenäquivalente einsetzt.

Vorzugsweise setzt man bei dem erfindungsgemässen Verfahren als N-Cyano-N-(ar)alkyl- oder N-Cyano-N-(meth)allylcarbonsäureamid Verbindungen der Formel II ein, insbesondere solche, worin R ein Alkyl mit 1 bis 4 C-Atomen oder Benzyl und $R^2$ ein Alkyl mit 1 bis 4 C-Atomen bedeuten.

Die Alkylgruppe R kann geradkettig oder verzweigt sein. solcher Alkylgruppen seien genannt: Methyl, Aethyl, n-Propyl, Isopropyl, n-Butyl sek.-Butyl n-Pentyl, 2-Pentyl, n-Hexyl, n-Heptyl, 3-Heptyl und n-Octyl.

Die Aralkylgruppe $R^1$ bedeutet vorzugsweise 2-Phenylathyl oder Benzyl, insbesondere Benzyl.

Die Alkylgruppe R kann geradkettig oder verzweigt, unsubstituiert oder substituiert sein. Als Substituenten eignen sich beispielsweise Chlor-, Bromatome oder Nitrogruppen, aber auch Alkoxygruppen mit 1 bis 4 C-Atomen, insbesondere die Methoxygruppe. Die Cycloalkyl- und Arylgruppe $R^2$ können gegebenenfalls auch solche Substituenten aufweisen.

Der Rest $R^3$, der sich von einer aliphatischen Dicarbonsäure ableitet, weist vorzugsweise 1 bis 8 C-Atome auf und stellt beispielsweise den entsprechenden Rest der Bernsteinsäure, Glutarsäure, Adipinsäure oder Sebacinsäure dar.

Die Verbindungen der Formel II sind zum Teil in der Literatur bereits beschrieben worden und können vorteilhaft hergestellt werden, indem man 1 Mol eines N-Cyancarbonsäureamidsalzes der Formel IV

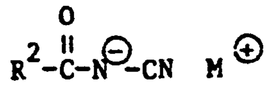 (IV),

worin $R^2$ die gleiche Bedeutung wie in Formel II hat und $M^{\oplus}$ für ein Na- oder K-Kation steht, mit 1 Mol eines Alkylierungsmittels, wie Alkylhalogenid, Dimethyl- oder Diäthylsulfat, in einem polaren aprotischen Lösungsmittel umsetzt.

Desgleichen können die Verbindungen der Formel III, die in der Literatur noch nicht beschrieben wurden, hergestellt werden, indem man 1 Mol eines Dicarbonsäuredicyandiamidsalzes der Formel V

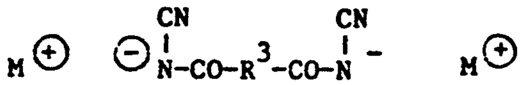 (V),

worin $R^3$ die gleiche Bedeutung wie in Formel III hat und $M^{\oplus}$ für ein Na- oder K-Kation steht, mit 1 Mol der zuvorgenannten Alkylierungsmittel umsetzt.

Die Durchführung des erfindungsgemässen Verfahrens mit wässriger Natron-,Kalilauge oder Ammoniaklösung ist bezüglich der Konzentration dieser Lösungen nicht kritisch. Vorzugsweise verwendet man 1 bis 50 gew.-%ige wässrige Natron- oder Kalilauge bzw. 1 bis 50 vo1.-%ige wässrige Ammoniaklösungen. Es hat sich ferner gezeigt, dass es von Vorteil ist, dem aus einer Verbindung der Formel II oder III und der wässrigen Lauge bestehenden Reaktionsgemisch zur besseren Benetzung ein wenig Alkohol, wie beispielsweise Aethanol oder Propanol, zuzugeben.

Als primäre aliphatische oder cycloaliphatische Amine können beim erfindungsgemässen Verfahren sowohl Monoamine als auch Polyamine mit mindestens einer primären Aminogruppe eingesetzt werden.

Beispielsweise eignen sich:.

Methylamin, Allylamin, Butylamin, iso-Butylamin, Hexylamin, Cyclohexylamin, Aminomethylcyclohexan, Benzylamin, Aethanolamin, 3-Propanolamin, Aethylendiamin, Tetramethylendiamin, Hexamethylendiamin, 2,2-Dimethyl-1,3-diaminopropan, 2,5-Dimethyl-1,5-diaminoheptan, 2,5-Dimethyl-1,6-diaminohexan, 2,5-Dimethyl-1,7-diaminoheptan, 3,3,5-Trimethyl-1,6-diaminohexan, 1,2-Bis-(3-aminopropoxy)-äthan, 3-Methoxy-1,6-diaminohexan, $H_2N(CH_2)_3O(CH_2)_3NH_2$, $H_2N(CH_2)_3S(CH_2)_3NH_2$, $H_2N$-$C_2H_4$-S-$C_2H_4$-$NH_2$, $H_2N(CH_2)_3N(CH_3)(CH_2)_3NH_2$, N,N'-Bis-(3-aminopropyl)-5,5-dimethylhydan-toin, 4,4'-Diaminodicyclohexylmethan, 3,3'-Dimethyl-4,4'-diaminodicyclohexylmethan, 4,4'-Diaminodicyclohexyläther, 4,4'-Diaminodicyclo-hexylsulfon, 4,4'-Diaminodicyclohexylisopropan, 1,4-Bis-(aminomethyl)-cyclohexan, Diäthylentriamin, Triäthylentetramin, 3-Diäthylaminopropylamin, $[(CH_3)_2CHO]_2PO(CH_3)_2NH_2$,

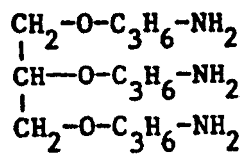,

$CH_3$-$CH_2$-$C(CH_2$-O-$C_3H_6$-$NH_2)_3$

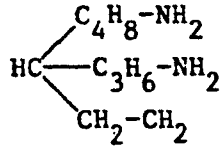

und 1,8-Diamino-4-aminomethyloktan.

Vorzugsweise verwendet man als Amine primäre aliphatische Mono- oder Diamine.

Bei der Umsetzung der primären aliphatischen oder cycloaliphatischen Amine mit den Verbindungen der Formel II oder III kann als Lösungsmittel sowohl Wasser als auch ein organisches Lösungsmittel verwendet werden. Geeignete organische Lösungsmittel sind dafür zum Beispiel cycloaliphatische oder aromatische Kohlenwasserstoffe, wie Cyclohexan, Benzol, Toluol und Xylole; aliphatische oder cyclische Aether, wie Diäthyläther, Dioxan und Tetrahydrofuran; chlorierte aliphatische Kohlenwasserstoffe, wie Methylenchlorid,

Chloroform, Tetrachlormethan, Trichloräthylen und Dichloräthan; Aethylenglykoldimethyl- und -diäthyläther; Alkanole, wie Methanol, Aethanol, n-Propanol, Isopropanol und Butanol; aliphatische Ketone, wie Aceton; cyclische Amide, wie N-Methylpyrrolidon; Ni-Dialkylamide von niederen aliphatischen Monocarbonsäuren, wie N,N-Dimethylformamid und N,N-Dimethylacetamid; Dialkylsulfoxide, wie Dimethyl- und Diäthylsulfoxid; Hexamethylphosphorsäuretriamid und Sulfolan; Alkylnitrile mit 2-5 C-Atomen, wie Acetonitril, Propionitril und Butyronitril; cyclische Amine, wie Pyridin und seine Derivate.

Die Konzentrationen der Verbindungen der Formel 11 und 111 und des primären aliphatischen oder cycloaliphatischen Amins im Lösungsmittel können innerhalb weiter Grenzen variieren und liegen zweckmässig zwischen 10 und 50 Gewichts-%, vorzugsweise zwischen 20 und 40 Gewichts-%.

Bei der Durchführung des erfindungsgemässen Verfahrens mittels primären aliphatischen Alkoholen können als solche sowohl Mono- als auch Polyole eingesetzt werden. Als Polyole können zum Beispiel Di-, Tri- oder Tetrole verwendet werden. Diese können einen geradkettigen oder verzweigten, ungesättigten oder gesättigten, gegebenenfalls mit Aethersauerstoffatomen unterbrochenen oder mit Halogenatomen, Alkoxy, oder Phenoxy substituierten aliphatischen Rest enthalten. Als Beispiele solcher Verbindungen seien genannt: Methanol, Aethanol, Propanol, Aethylenglykol, Propandiol-1,3, Hexandiol-1,6, Neopentylglykol, 2-Aethylhexandiol-1,3, Butandiol-1,4, Diäthylenglykol, Dipropylenglykol, 1,1,1-Trimethyloläthan, 1,1,1-Trimethylolpropan, Glycerin, Hexantriol-1,2,6 und Pentaerythrit.

Vorzugsweise verwendet man als Alkohol ein aliphatisches Mono- oder Diol.

Als Katalysatoren eignen sich im Prinzip alle basischen Verbindungen, wie beispielsweise Natron-, Kalilauge, Ammoniak, primäre, sekundäre oder tertiäre Amine, wie Methylamin, Butylamin, Benzylamin, Diäthylamin, Tributylamin oder Triäthylamin, quaternäre Ammoniumbasen, wie Benzyltrimethylammoniumhydroxid, heterocyclische Basen, wie Pyridin, Chinolin, N-Methylpyrrolidon, Imidazol und deren Derivate, und die Alkalialkoholate, wie Natriummethylat.

Das erfindungsgemässe Verfahren, das in einem breiten Temperaturbereich durchgeführt werden kann, wird vorzugsweise im Temperaturberech von 0 bis 120° C durchgeführt. Wie eingangs erwähnt, stellen Isomelamine Modifizierungsmittel für Polyester dar. Durch Hydrolyse von Trialkylisomelaminen erhält man ausserdem die entsprechenden Trialkylcyanursäuren.

Es wurde nun ferner gefunden, dass die erfindungsgemäss hergestellten Isomelamine wertvolle Härtungsmittel für Epoxidharze darstellen. Gegenstand der vorliegenden Erfindung ist somit auch ein Verfahren zum Härten von Epoxidharzen, indem man die Verbindungen der Formel II oder III gemäss dem erfindungsgemässen Verfahren umsetzt und inden man die so erhaltenen Isomelamine der Formel I mit Epoxidharzen vermischt.

Bei dem Verfahren zum Härten von Epoxidharzen wird die Menge des als Härtungsmittel eingesetzten Isomelamins so berechnet, dass normalerweise 0,75 bis 1,25 NH-Aequivalente des Isomelamins auf 1 Epoxidäquivalent kommen. Vorzugaweise verwendet man das Isomelamin und die Epoxidharzkomponente in gleichen äquivalenten Mengen.

Beim Härten von Epoxidharzen sind vorzugsweise solche Epoxidharze verwendbar, welche direkt an Sauerstoff-, Stickstoff- oder Schwefelatome gebundene Cruppen der Formel IV

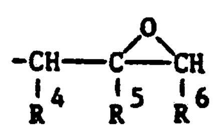

(IV)

enthalten, worin entweder $R^4$ und $R^6$ je ein Wasserstoffatom darstellen, in welchem Fall $R^5$ ein Wasserstoffatom oder eine Methylgruppe bedeutet, oder $R^4$ und $R^6$ zusammen $-CH_2CH_2-$darstellen, in welchem Fall $R^5$ ein Wasserstoffatom bedeutet.

Als Beispiele solcher Harze seien Polyglycidyl- und Poly-(ß-methylglycidyl)-ester genannt, die man durch Umsetzung einer zwei oder mehr Carbonsäuregruppen pro Molekül enthaltenden Verbindung mit Epichlorhydrin, Glycerindichlorhydrin oder ß-Methylepichlorhydrin in Gegenwart von Alkali erhalten kann. Solche Polyglycidylester können sich von aliphatischen Polycarbonsäuren, z.B. Oxalsäure, Bernsteinsäure, Glutarsäure, Adipinsäure, Sebacinsäure oder dimerisierter oder trimerisierter Linolsäure, von cycloaliphatischen Polycarbonsäuren, wie Tetrahydrophthalsäure, 4-Methyltetrahydrophthalsäure, Hexahydrophthalsäure und 4-Methylhexahydrophthalsäure, und von aromatischen Polycarbonsäuren, wie Phthalsäure, Isophthalsäure und Terephthalsäure, ableiten.

Weitere Beispiele sind Polyglycidyl- und poly(ß-methylglycidyl)-äther, die durch Umsetzung einer mindestens zwei freie alkoholische und/oder phenolische Hydroxylgruppen pro Molekül enthaltenden Verbindung mit dem entsprechenden Epichlorhydrin unter alkalischen Bedingungen, oder auch in Gegenwart eines sauren-Katalysators mit nachfolgender Alkalibehandlung, erhältlich sind. Diese Aether lassen sich mit Poly-(epichlorhydrin) aus acyclischen Alkoholen, wie Aethylenglykol, Diäthylenglykol und höheren Poly-(oxyäthylen)-glykolén, Propan-1,2-diol und Poly-(oxypropylen)-glykolen, Propan-l,3-diol, Butan-1,4-diol, Hexan-2,4,6-triol, Glycerin, 1,1,1-Trimethylolpropan, Pentaerythrit und Sorbit, aus cycloaliphatischen Alkoholen, wie Resorcit, Chinit, Bis-(4-hydroxycyclohexyl)-methan, 2,2-Bis-(4-hydroxycyclohexyl)-propan und 1,1-Bis-(hydroxymethyl)-cyclohexen-3, und aus Alkoholen mit aromatischen Kernen, wie N,N-Bis-(2-hydroxyäthyl)-anilin und p,p'-Bis-(2-hydroxyäthylamino)-diphenylmethan, herstellen. Ferner sind auch Epoxidharze aus einkerniden Phenolen, wie Resorcin und Hydrochinon, und mehrkernigen Phenolen, wie Bis-(4-hydroxyphenyl)-methan, 4,4'-Di-hydroxydiphenyl, Bis-(4-hydroxyphenyl)-sulfon, 1,1,2,2-Tetrakis-(4-hydroxyphenyl)-äthan, 2,2-Bis-(4-hydroxyphenyl)-propan (Bisphenol A) und 2,2-Bis-(3,5-dibrom-4-hydroxyphenyl)-propan sowie aus Aldehyden, wie Formaldehyd, Acetaldehyd, Chloral und Furfurol, mit Phenolen, wie Phenol selbst und durch Chloratome oder Alkylgruppen mit jeweils bis zu neun Kohlenstoffatomen ringsubstituiertem Phenol, wie 4-Chlorphenol, 2-Methylphenol und 4-tert.Butylphenol, gebildeten Novolaken geeignet.

Weitere geeignete Poly-(N-glycidyl)-Verbindungen umfassen beispielsweise solche, die durch Dehydrochlorierung der Umsetzungsprodukte von Epichlorhydrin mit miniestens zwei Aminowasserstoffatome enthaltenden Aminen wie Anilin, n-Butylamin, Bis-(4-aminophenyl)-methan und Bis-(4-methylaminophenyl)-methan, erhalten werden, sowie Triglycidylisocyanurat oder N,N'-Diglycidylderivate von cyclischen Alkylenharnstoffen, wie Aethylenharnstoff und 1,3-Propylenharnstoff, oder Hydantoinen, wie 5,5-Dimethylhydantoin.

Beispiele für Epoxidharze mit Gruppen der Formel IV, wor n $R^4$ und $R^6$ zusammen eine $-CH_2CH_2$-Gruppe bedeuten, sind Bis-(2,3-epoxycyclopentyl)-äther, 2,3-Epoxycyclopentyl-glycidyläther und 1,2-Bis-(2,3-epoxycyclopentyloxy)-äthan.

Ebenfalls einsetzbar sind auch Epoxidharze, in denen einige oder sämtliche Epoxidgruppen mittelständig sind, wie Vinylcyclohexendioxyd, Limonendioxyd, Dicyclopentadiendioxyd, der 3,4-Epoxycyclohexylmethylester der 3',4'-Epoxycyclohexancarbonsäure sowie dessen 6,6'-Dimethyl-derivat, der Bis-(3,4-epoxycyclohexancarbonsäureester) des Aethylenglykols, Adipinsäure-bis-(3,4-epoxy-6-methylcyclohexylester) und 3-(3,4-Epoxycyclohexyl)-8,9-epoxy-2,4-dioxaspiro[5,5]undecan.

Die härtbaren Mischungen können ferner noch Plastifizierungsmittel, wie Dibutylphthalat, Dioctylphthalat oder Trikresylphosphat, oder Additive enthalten, wie Füllstoffe, Verkstärkungsmittel, Färbemittel, Fliessmittel, flammhemmende Stoffe und Formtrennmittel. Geeignete Streckmittel, Füllstoffe und Verstärkungsmittel sind beispielsweise Asbest, Asphalt, Bitumen, Glasfasern, Textilfasern, Kohlenstoff- oder Borfasern, Glimmer, Tonerde, Gips, Titandioxid, Kreide, Quarzmehl, Cellulose, Kaolin, gemahlener Dolomit, Wollastonit, Kieselerde mit grosser spezifischer Oberfläche (erhältlich unter dem Handelsnamen "Aerosil"), mit langkettigen Aminen modifizierte Tonerde (erhältlich unter dem Handelsnamen "Bentone"), gepulvertes Poly(vinylchlorid), Polyolefin oder Aminoplast, Metallpulver, wie Aluminium- oder Eisenpulver. Flammschutzmittel, wie Antimontrioxid, können ebenfalls den härtbaren Mischungen zugegeben werden.

Die folgenden Beispiele beschreiben die Erfindung näher. Wenn nichts anderes vermerkt ist, bedeuten Teile Gewichtsteile.

**Beispiele 1-14: 1,3,5-Trimethylisomelamin**

1. 5 19 g (0 0529 Mol) N-Cyano-N-methylacetamid werden mit einer Lösung von 3,0 g KOH (0,0536 Mol) in 20 ml $H_2O$ versetzt, wobei zur besseren Benetzung 1 ml Aethanol zugegeben wird. Das Gemisch reagiert exotherm und bildet eine klare Lösung. Man erhitzt kurz zum Sieden; beim Erkalten kristallisiert das Produkt aus. Man kühlt über Nacht im Eisschrank, saugt ab, wäscht mit kaltem Wasser und trocknet bei 70°C im Vakuumtrockenschrank. Ausbeute 2,16 g (72,8% der Theorie), Schmelz punkt: 175-177°C (Literatur: 178°C s1) 2) 3)). Lässt man die so getrocknete $H_2O$-freie Verbindung 6 Tage offen an der Luft stehen, so nimmt sie 3 Mol Kristallwasser auf und schmilzt unter Abgabe desselben, wenn eine Probe bei 150°C in den Schmelzblock gebracht wird.

Analyse für $C_6H_{12}N_6.3H_2O$

berechnet: $H_2O$ = 24,32%, gefunden: $H_2O$ = 23,83%

1) R. Kitawaki, J. Org. Chem. 25, 1043 (1960)
2) A.W. Hofmann, Chem. Ber. 3, 264
3) A.W. Hofmann, Chem. Ber. 18,

Das $^{13}C$-NMR-Spektrum bestätigt die Struktur für 1,3,5-Trimethylisomel-amin: 148,4 ppm (Ring C; Septett); 31,5 ppm ($CH_3$; Quartett).

2. Man löst 2,52 g (0,030 Mol) N-Cyano-N-methylformamid in 5 ml Aethanol, fügt 1,85 g (0,033 Mol) KOH gelöst in 5 ml $H_2O$ zu und erhitzt kurz zum Sieden. Beim Erkalten kristallisiert viel Produkt aus. Man verdünnt mit weiteren 10 ml $H_2O$, löst nochmals durch Erhitzen und lässt bei Raumtemperatur auskristallisieren. Absaugen, waschen mit $H_2O$ und trocknen bei 50°C im Vakuum gibt 0,92 g (84 7% d Th ) Produkt vom Schmelzpunkt 180°C. Durch Einengen des Filtrats erhält man weitere 0,14 g (8,32) vom Schmelzpunkt 179-180°C.

3. 3,20 g (0,020 Mol) N-Cyano-N-methylbenzamid werden in 5 ml Aethanol gelöst und mit einer Lösung von 1,23 g KOH (0,022 Mol) in 3 ml $H_2O$ versetzt. Man erhitzt kurz zum Sieden; beim Erkalten kristallisiert das Produkt aus. Man kühlt im Eisschrank, saugt ab, wäscht mit kaltem 402igem Aethanol und trocknet bei 60°C im Vakuum. Ausbeute 0,5 g (48,22 d.Th.), Schmelzpunkt 177-179°C.

4. 4,76 g (0,020 Mol) N-Cyano-N-methyllaurinsäureamid werden in 10 ml warmem Aethanol gelöst und mit einer Lösung von 1,23 g (0,022 Mol) KOH in 2 ml $H_2O$ versetzt. Man erhitzt kurz zum Sieden und kühlt dann im

Eisschrank. Absaugen, Waschen mit kaltem Aethanol und Trocknen bei 60°C im Vakuum ergibt 0,44 g (392 d.Th.)-Produkt vom Schmelzpunkt 180°C.

5. 13 33 g (0,060 Mol) N,N-Dicyano-N,N'-dimethyladipinsäurediamid werden mit 1 ml Triäthylamin in 20 ml (0,268 Mol) n-Propanol über Nacht im Gelbad von 100°C gerührt. Beim Erkalten kristallisiert Produkt aus, das nach Zugabe von 20 ml Aceton abgesaugt, mit Aceton gewaschen und dann getrocknet wird. Ausbeute 2,91 g (86,62 d.Th.); Schmelzpunkt 179°C Einengen des Filtrats gibt weiteres Festprodukt vermischt mit etwas harzigen Anteilen. Letztere lösen sich mit Aceton.

Man saugt ab, wäscht mit Aceton, trocknet und erhält weitere 0,89 g unreines Produkt. Beide Fraktionen werden zusammen aus der 10-fachen Menge $H_2O$ (37 ml) umkristallisiert, kalt abgesaugt, mit kaltem $H_2O$ gewaschen und bei 60°C im Vakuum getrocknet. Ausbeute 3,13 g (93 0% d.Th.); Schmelzpunkt 178-179°C.

6. 2,94 g (0,03 Mol) N-Cyano-N-methylacetamid werden in 12 ml Tetrahydrofuran mit 3,21 g (0,03 Mol) Benzylamin 8 Stunden am Rückfluss gekocht. Das Produkt kristallisiert beim Erkalten aus. Man saugt ab, wäscht mit Tetrahydrofuran und trocknet bei 80°C im Vakuum. Ausbeute 0,52 g; Schmelzpunkt 173-176°C. Das Filtrat wird eingeengt und der Rückstand aus einem Gemisch von 15 ml $H_2O$ und 3 ml Aethanol umkristallisiert. Man erhält weitere 0,56 g Produkt vom Schmelzpunkt 176-178°C. Gesamtausbeute: 1,08 g (68,72 d.Th.).

7. 2,52 g (0,03 Mol) N-Cyano-N-methylacetamid werden in 7 ml $H_2O$ mit 3 ml 25%igem wässerigem Ammoniak (0,044 Mol) versetzt. Das Produkt löst sich unter eigener Erwärmung. Man erhitzt kurz zum Sieden, wonach das Produkt beim Erkalten auskristallisiert. Absaugen, waschen mit kaltem Wasser und trocknen bei 60°C im Vakuum ergibt 0,94 g (55,92) Trimethylisomelamin.

8. 4 90 g (0,05 Mol) N-Cyano-N-methylacetamid werden in 15 ml (0,2 Mol) n-Propanol gelöst, mit 0,3 ml Triäthylamin versetzt und über Nacht in ein Oelbad von 85°C getaucht. Beim Erkalten kristallisiert das Produkt aus. Absaugen, waschen mit n-Propanol und trocknen bei 70°C im Vakuum ergibt 1,44 g Produkt vom Schmelzpunkt 170-173°C. Einengen des Filtrats, Umkristallisation des Rückstands aus 5 ml Wasser und Trocknung bei 60°C im Vakuum gibt weitere 0,87 g Produkt vom Schmelzpunkt 170-173°C. Gesamtausbeute an roher Verbindung 2,31 g (82,4%).

9. 5 67 g (0,0579 Mol) N-Cyano-N-methylacetamid werden mit einer Lösung von 4,87 g (0,087 Mol) KOH in 10 ml $H_2O$ versetzt und erhitzt. Nach 2 Minuten lässt man erkalten, wobei das Produkt auskristallisiert. Nach dem Absaugen, waschen mit $H_2O$ und Trocknen bei 60°C im Vakuum erhält man 1,42 g (43,7% d.Th.) 1,3,5-Trimethylisomelamin.

10. 2,94 g (0,030 Mol) N-Cyano-N-methylacetamid werden in 7,2 g (0,12 Mol) n-Propanol und 0,22 g Diäthylamin 21 Stunden am Rückfluss gekocht. Die klare Lösung kristallisiert beim Erkalten. Absaugen, Waschen mit n-Propanol und Trocknen bei 60° im Vakuum ergibt 0,68 g (40,42) Produkt vom Schmelzpunkt 178-180°C. Aus dem Filtrat können durch Einengen weitere 0,45 g (26,8% d.Th.) Produkt vom Schmelzpunkt 175-179°C isoliert werden.

11. 2,94 g (0,030 Mol) N-Cyano-N-methylacetamid werden mit 0,64 g 1-Dimethylaminododecan in 15 ml (0,164 Mol) n-Butanol gelöst und 17 Stunden in ein Bad von 100°C getaucht. Beim Erkalten kristallisiert Produkt aus, das abgesaugt, gewaschen und getrocknet wird. Ausbeute 0,59 g. Einengen der Mutterlauge liefert weitere 0,36 g. Die Gesamtausbeute beträgt somit 0,95 g (56,5% d.Th.); Schmelzpunkt: 176-180°C.

12. 2,95 g (0,030 Mol) N-Cyano-N-methylacetamid werden mit 0,35 g N,N,N',N'-Tetramethyläthylendiamin in 20 ml (0,105 Mol) Decanol warm gelöst und 18 Stunden in ein Bad von 100°C getaucht. Das Produkt kristallisiert beim Erkalten, und in der flüssigen Phase lässt sich gaschromatographisch kein Edukt mehr nachweisen. Man fügt Isopropanol zu, saugt ab, wäscht mit Isopropanol und trocknet bei 60°C im Vakuum. Ausbeute: 0,50 g; Schmelzpunkt: 180-181°C. Das Filtrat wird eingeengt und nach vollständiger Entfernung von überschüssigem n-Decanol durch Vakuumdestillation mit 5 ml $H_2O$ und 2,5 ml Aethanol versetzt. Bei Erhitzen entsteht eine klare Lösung, aus der weitere 0,37 g produkt vom Schmelzpunkt 176-179°C auskristallisieren. Gesamtausbeute 0,87 g (51,7% d.Th.).

13. 2,94 g (0,030 Mol) N-Cyano-N-methylacetamid werden mit 5,59 g (0,090 Mol) Aethylenglykol und 0,30 g (0,003 Mol) Triäthylamin in 5 ml Tetrahydrofuran am Rückfluss über Nacht gekocht. Danach werden alle bis 140°C bei 26 mbar siedenden Anteile entfernt und der Rückstand aus 12 ml $H_2O$ umkristallisiert. Ausbeute 0,89 g (52,9 % d.Th.); Schmelzpunkt 179-181°C.

14. 2,94 g (0,030 Mol) N-Cyano-N-methylacetamid werden mit 2 34 g (0,016 Mol) Triäthylentetramin in 15 ml Tetrahydrofuran 5 Stunden gekocht; beim Erkalten kristallisieren 0,70 g Produkt vom Schmelzpunkt 179-181°C aus. Durch Einengen der Mutterlauge erhält man weitere 0,48 g Substanz vom Schmelzpunkt 178-181°C; die Gesamtausbeute beträgt also 1,18 g (70,2% d.Th.).

**Beispiele 15 und 16: 1,3,5-Triäthylisomelamin**

15. 2,24 g (0,02 Mol) N-Cyano-N-äthylacetamid werden bei Raumtemperatur in einem Gemisch von 2 ml Aethanol und 3 ml $H_2O$ gelöst und mit einer Lösung von 1,3 g (0,0232 Mol) KOH in 3 ml $H_2O$ versetzt. Es scheidet sich ein Oel ab, das sich infolge exothermer Reaktion wieder löst. Man kühlt über Nacht im Eisschrank, saugt ab, wäscht mit kaltem Wasser und trocknet bei 60°C im Vakuum. Ausbeute: 0,89 g (63,6% d.Th.); Schmelzpunkt: 91-92°C (Literatur: 3) 92°C). Durch Einengen des Filtrats erhält man weitere 0,24 g (17,1%) vom Schmelzpunkt 89-91°C. Beide Fraktionen werden zusammen nochmals aus 7 ml $H_2O$ umkristallisiert, nach 6-

stündigem Kühlen im Eisschrank abgesaugt, mit kaltem Wasser gewaschen und bei 40°C im Vakuum getrocknet. Ausbeute 1,10 g (78 6% d. Th.); Schmelzpunkt 91-92°C. Das Produkt nimmt beim offenen Stehen an der Luft kein Kristallwasser auf; $H_2O$-Analyse: Gehalt

$<$0,3% $H_2O$. Das

$^{13}C$-NMR-Spektrum bestätigt die Struktur: 146,6 ppm (Ringe C; Quintett); 39,3 ppm ($CH_2$); 11,9 ppm ($CH_3$).

16. 4,49 g (0,04 Mol) N-Cyano-N-äthylacetamid werden mit 15 ml (0,2 Mol) n-Propanol und 0,3 ml Triäthylamin 24 Stunden in ein Oelbad von 110°C getaucht, wonach sich gaschromatographisch kein Edukt mehr nachweisen lässt. Man engt am Rotationsverdampfer ein und erhält ein bei Raumtemperatur kristallin erstarrendes Oel. Die Umkristallisation aus 6 ml $H_2O$ ergibt nach dem Absaugen, dem Waschen mit kaltem $H_2O$ und dem Trocknen im Vakuum bei 35°C 1,28 g (45% d.Th.) Substanz vom Schmelzpunkt 90-91°C.

**Beispiel 17: 1,3,5-Triallylisomelamin**

7 44 g (0,06 Mol) N-Cyano-N-allylacetamid werden mit einer Lösung von 3,4 g (0,0607 Mol) KOH in 10 ml $H_2O$ versetzt. Nach Zugabe von 6 ml Aethanol wird das Gemisch homogen und reagiert exotherm. Nach kurzer Zeit scheidet sich ein Oel ab, das sich durch Zugabe von 3 ml Aethanol und kurzes Aufkochen in Lösung bringen lässt. Nach dem Erkalten wird mit 10 ml $H_2O$ verdünnt und mit 3 portionen Chloroform (20, 10 und 10 ml) extrahiert. Einengen der Extrakte am Rotationsver-dampfer ergibt 4,77 g eines farblosen Oels, das zur Reinigung im Kugelrohrofen destilliert wird. Man erhält 3,54 g (71,92 d.Th.) Destillat vom Siedpunkt 105-115°C bei 0,026 mbar, das bei Raumtemperatur kristallin erstarrt und gaschromatographisch zu über 97% rein ist.

Analyse $C_{12}H_{18}N_6$ (M = 246,32):

| | | | |
|---|---|---|---|
| berechnet: | C 58,52% | gefunden: | C 58,32% |
| | H 7,37% | | H 7,34% |
| | N 34,12% | | N 34,57%. |

**Beispiel 18: 1,3,5-Tri-n-butylisomelamin**

12,60 g (0,09 Mol) N-Cyano-N-butylacetamid werden mit 13 4 g (0 1035 Mol) Diäthylamino-propylamin in 25 ml Tetrahydrofuran 2 Stunden am Rückfluss gekocht, wonach nach gaschromatographischer Untersuchung das Edukt vollständig reagiert hat zu 3-Diäthylaminopropylacetamid, Tri-n-butylisomelamin und einer weiteren Komponente, die wahrscheinlich aus Butylcyanamid besteht. Man engt am Rotationsverdampfer ein, lässt das zurückbleibende Oel bei Raumtemperatur stehen und verfolgt die weiter stattfindende Reaktion gaschromatographisch. Nach 5 Tagen hat sich die unbekannte Komponente, die wahrscheinlich aus Butylcyanamid besteht, weitgehend umgesetzt, und durch Trimerisation Tributylisomelamin gebildet. Das Gemisch wird im Kugelrohrofen zunächst bei 23,4 mbar und maximal 125°C destilliert, danach bei 0,156 mbar und maximal 180°C. Man erhält dabei 6 Fraktionen von zusammen 24,4 g, deren gaschromatographische Analyse eine Ausbeute von 74,7% an Tributylisomelamin und 96,7% an 3-Diäthylaminopropylacetamid ergibt. Durch erneute Destillation der im Hochvakuum erhaltenen Fraktionen lässt sich eine Analysenprobe des bei Raumtemperatur flüssigen Tributylisomelamins isolieren.

Analyse $C_{15}H_{30}N_6$ (M = 294,45)

| | | | |
|---|---|---|---|
| berechnet: | C 61,19% | gefunden: | C 61,15% |
| | H 10,27% | | H 10,12% |
| | N 28,54% | | N 28,69% |

$^{13}C$-NMR-Spektrum (ppm): 147,2 (Ring-C); 44,1 ($NCH_2$); 28,7 ($CH_2$); 20,2 ($CH_2$); 13,8 ($CH_3$).

Mit Pikrinsäure erhält man aus Aethanol das Dipikrat vom Schmelzpunkt 179-181°C.

Analyse $C_{15}H_{30}N_6 \cdot 2C_6H_3N_3O_7$ (M = 752,66)

| | | | |
|---|---|---|---|
| berechnet: | C 43,09% | gefunden: | C 42,94% |
| | H 4,82% | | H 4,80% |
| | N 22,33% | | N 22,43% |
| | O 29,76% | | O 30,04%. |

**Beispiel 19: 1,3,5-Tri-isobutylisomelamin**

3,25 g (0,0232 Mol) N-Cyano-N-isobutylacetamid werden mit 10 ml Aethanol und 1,55 g (0,0277 Mol) KOH, gelöst in 10 ml $H_2O$, 60 Minuten lang gekocht. Danach wird am Rotationsverdampfer eingeengt, mit 10 ml $H_2O$ versetzt und mit Methylenchlorid extrahiert. Nach dem Entfernen des Lösungsmittels erhält man 1,40 g Rohprodukt, das bei Raumtemperatur kristallin erstarrt. Nan destilliert im Kugelrohrofen bei 80-110°C und 1,3.10 mbar und erhält 0,78 geinesbeiRaumtemperatur erstarrenden Oels. Umkristallisation aus einem Gemisch von n-Hexan und Benzol ergibt 0,30 g reines Triisobutylmelamin vom Schmelzpunkt 91-93°C.

Analyse $C_{15}H_{30}N_6$ (N = 294,45)
berechnet C 61,192 gefunden C 60,97%
H 10,27 2 H 10,122
N 28,542 N 28,482
$^{13}$C-NNR-Spektrum (ppm): 148,2 (Ring-C); 51,0 ($NCH_2$); 26,8 (CH); 20,2 ($CH_3$).

**Beispiel 20: 1,3,5-Tribenzylisonelamin**

52,2 g (0,30 Mol) N-Cyano-N-benzylacetamid werden in 150 ml Tetrahydrofuran mit 44,9 g (0,3448 Mol) 3-Diäthylaminopropylamin 2 L/2 Stunden am Rückfluss gekocht. Nan engt am Rotationsverdampfer ein, kristallisiert den-Rückstand von 101 g aus 300 ml Cyclohexan um und erhält 28,2 g produkt vom Schmelzpunkt 124-126°C. Einengen der Mutterlauge und Umkristallisation aus 300 ml n-Hexan ergibt weitere 4,60 g Produkt vom Schmelzpunkt 119-121°C. Die Gesamtausbeute beträgt somit 32,8 g (82,82 d.Th.). Zur weiteren Reinigung wird nochmals aus einem Gemisch von 100 ml Cyclohexan und 66 ml Toluol umkristallisiert. Ausbeute 28,7 g; Schmelzpunkt 129-129,5°C (Literatur 4)): 129-130°C).

4) L.Birkofer, Chem. Ber.75, 429.

**Anwendundsbeispiele I-IV**

Flüssiger Bisphenol-A-diglycidyläther mit einem Epoxidgehalt von 5,35 Aequivalenten/kg und Trialkylisomelamin werden in solchen Verhaltnissen auf einem Dreiwalzenstuhl bei Raumtemperatur gemischt, dass auf jedes NH-Aequivalent des Isomelamins ein Epoxidgruppenäquivalent kommt. Diese Suspensionen werden dann weiter untersucht.

a) Glasumwandlungstemperatur (GUT):.

4 g der Suspension werden in einen dünnwandigen Aluminiuntiegel gegeben und ausgehärtet. An Proben des gehärteten Materials werden mittels der Differential-Thermoanalyse (Gerät "TA 2000" der Firma Mettler, Greifensee, CH,) die Werte der GUT ermittelt.

b) Zugfestigkeit:

Eine kleine Menge der Suspension wird auf die Enden von Prüfstreifen aus Anticorodalblech (Aluminiumlegierung), das vorher durch Schleifen aufgerauht und mit Lösungsmitteln entfettet worden ist, aufgetragen. Die Abmessungen der Streifen betragen 170 x 25 x 15 mm. Man lässt die Streifen 12 mm überlappen, fixiert mit einer Klemme und härtet aus. Danach wird die Zugscherfestigkeit nach DIN 53 183 ermittelt.

Die Werte sind in der folgenden Tabelle zusammengefasst.

| | Anwendungsbeispiele | | | |
|---|---|---|---|---|
| | I | II | III | IV |
| 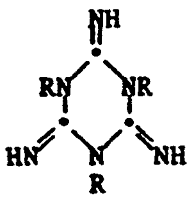 | R = $-CH_3$<br>30,5 g | R = $-C_2H_5$<br>38,2 g | R = $CH_2CH{=}CH_2$<br>44,7 g | R = $-CH_2C_6H_5$<br>71,9 g |
| Epoxidharz | 100 g | 100 g | 100 g | 100 g |
| Härtungsbedingung | 6 Stunden bei 180°C | | | |
| GUT | 95°C | 80°C | 93°C | 77°C |
| Zugscherfestigkeit [$N/mm^2$] | 9,9 | 19,0 | 16,5 | 9,4 |

**Patentansprüche**

1. Verfahren zur Herstellung von Isomelaminen der Formel I

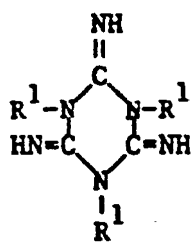 (I),

worin jedes $R^1$ für ein Alkyl mit 1 bis 8 C-Atomen, Aralkyl mit bis zu 12 C-Atomen Allyl oder Methallyl steht, dadurch gekennzeichnet, dass man N-Cyano-N-(ar)alkyl- oder N-Cyano-N-(meth)-allylcarbonsäureamide der Formel II oder III

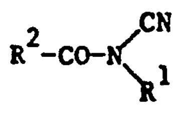 (II) oder

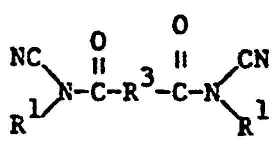 (III),

worin jedes $R^1$ die gleiche Bedeutung wie in Formel I hat, $R^2$ ein Wasserstoffatom, Alkyl mit 1 bis 16 C-Atomen, Cycloalkyl mit 5 bis 8 C-Atomen oder Aryl mit 6 oder 10 Ring-C-Atomen bedeutet und $R^3$ für eine direkte Bindung oder ein Alkylen mit 1 bis 12 C-Atomen steht, mit

a) wässriger Natron-, Kalilauge oder Amnoniaklösung oder

b) primären aliphatischen oder cycloaliphatischen Aminen in einem Lösungsmittel oder mit

c) primären aliphatischen Alkoholen in Gegenwart von katalytischen Mengen einer basischen Verbindung im Temperaturbereich von -20°C bis 200°C umsetzt, wobei man auf 3 Carbonsäureamidgruppenäquivalente mindestens 3 Hydroxyl- oder Aminogruppenäquivalente einsetzt.

2. Verfahren gemäss Anspruch 1, dadurch gekennzeichnet, dass man wässrige Natron- oder Kalilauge in Konzentrationen von 1 bis 50 Gewichts-% verwendet.

3. Verfahren gemäss Anspruch 1, dadurch gekennzeichnet, dass man ein primäres aliphatisches Mono- oder Diamin verwendet.

4. Verfahren gemäss Anspruch 1, dadurch gekennzeichnet, dass man ein aliphatisches Mono- oder Diol verwendet,

5. Verfahren gemäss Anspruch 1, dadurch gekennzeichnet, dass man als N-Cyano-N-(ar)alkyl- oder N-Cyano-N-(meth)allylcarbonsäureamide die Verbindung der Formel II einsetzt.

6. Verfahren gemäss Anspruch 1, dadurch gekennzeichnet, dass man Verbindungen der Formel II verwendet, worin $R^1$ eim Alkyl mit 1 bis 4 C-Atomen oder Benzyl und $R^2$ ein Alkyl mit 1 bis 4 C-Atomen bedeuten.

7. Verfahren gemäss Anspruch 1, dadurch gekennzeichnet, dass man die Umsetzungsreaktion im Temperaturbereich von 0 bis 120°C durchführt.

8. Verfahren zum Härten von Epoxidharzen, dadurch gekennzeichnet, dass man die Verbindungen der Formel 11 oder III gemäss Anspruch 1 umsetzt und dass man die so erhaltenen Isomelamine der Formel 1 mit Epoxidharzen vermischt.

**Claims**

1. A process for the preparation of an isomelamine of the formula I

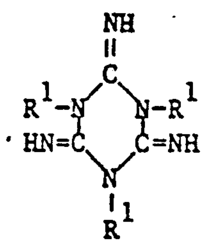

(I)

in which each R1 is alkyl having 1 to 8 C atoms, aralkyl having not more than 12 C atoms, allyl or methallyl, which comprises reacting an N-cyano-N-(ar)alkylcarboxamide or N-cyano-N-(meth)allylcarboxamide of the formula II or III

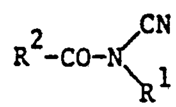

(II) or

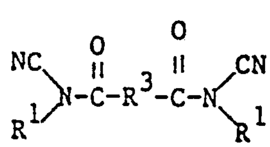

(III)

in which each $R^1$ is as defined under formula I, $R^2$ is a hydrogen atom, alkyl having 1 to 16 C atoms, cycloalkyl having 5 to 8 C atoms or aryl having 6 or 10 ring C atoms and $R^3$ is a direct bond or alkylene having 1 to 12 C atoms, with

a) an aqueous sodium, potassium or ammonia solution or

b) a primary aliphatic or cycloaliphatic amine in a solvent, or with

c) a primary aliphatic alcohol in the presence of a catalytic amount of a basic compound, in the temperature range from -20°C to 200°C, at least 3 hydroxyl or amino group equivalents being used per 3 carboxamide group equivalents.

2. A process according to claim 1, which comprises the use of aqueous sodium hydroxide solution or potassium hydroxide solution in a concentration of 1 to 50% by weight.

3. A process according to claim 1, which comprises the use of a primary aliphatic monoamine or diamine.

4. A process according to claim 1, which comprises the use of an aliphatic monool or diol.

5. A process according to claim 1, wherein the N-cyano-N-(ar)alkylcarboxamide or N-cyano-N-(meth)allylcarboxamide is a compound of the formula II.

6. A process according to claim 1, which comprises the use of a compound of the formula II in which $R^1$ is alkyl having 1 to 4 C atoms or benzyl and $R^2$ is alkyl having 1 to 4 C atoms.

7. A process according to claim 1, wherein the reaction is carried out in the temperature range from 0 to 120°C.

8. A process for curing an epoxy resin, which comprises reacting a compound of the formula II or III in accordance with the process as claimed in claim 1, and mixing the resultant isomelamine of the formula I with an epoxy resin.

**Revendications**

1. Procédé de préparation d'isomélamines répandant à la formule

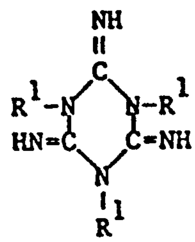

(I)

dans laquelle chacun des $R^1$ représente un radical alkyle contenant de 1 à 8 atomes de carbone, un radical aralkyle contenant au plus 12 atomes de carbone, un radical allyle ou un radical méthallyle, procédé caractérisé en ce qu'on fait réagir des N-cyano-N-(ar)alkyl-carboxamides ou des N-cyano-N-(méth)allyl-carboxamides répondant à l'une des formules II et III

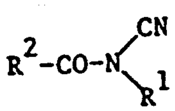

(II)

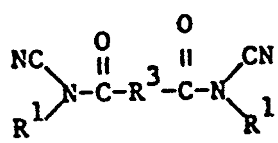

(III)

dans lesquelles $R^1$ ou chacun des $R^1$ a la même signification que dans la formule I, $R^2$ représente un atome d'hydrogène, un alkyle en $C_1$-$C_{16}$, un cycloalkyle en $C_5$-$C_8$ ou un aryle contenant de 6 à la atomes de carbone dans le cycle, et $R^3$ représente une liaison directe ou un radical alkylène contenant de 1 à 12 atomes de carbone, avec:

a) une solution aqueuse d'hydroxyde de sodium, d'hydroxyde de potassium ou d'ammoniac ou

b) des amines aliphatiques ou cycloaliphatiques primaires dans un solvant, ou avec

c) des alcools aliphatiques primaires en présence de quantités catalytiques d'un composé basique,

dans un intervalle de températures allant de -20 à 200°C, en mettant en jeu au moins 3 équivalents de radicaux hydroxy ou amino pour 3 équivalents de radicaux carbamoyles.

2. Procédé selon la revendication 1 caractérisé en ce qu'an utilise une solution aqueuse d'hydroxyde de sodium au d'hydroxyde de potassium dont la concentration est comprise entre 1 et 50 % en poids.

3. Procédé selon la revendication l'caractérisé en ce qu'on utilise une monoamine ou une diamine aliphatique primaire.

4. Procédé selon la revendication 1 caractérisé en ce qu'on utilise un monoalcool ou un diol aliphatique.

5. Procédé selon la revendication 1 caractérisé en ce qu'on utilise un composé de formule II comme N-cyano-N-(ar)alkyl-carboxamide un N-cyano-N-(méth)allylcarboxamide.

6. Procédé selon la revendication 1 caractérisé en ce qu'on utilise des composés de formule II dans lesquels $R^1$ représente un radical alkyle contenant de 1 à 4 atomes de carbone ou radical benzyle et $R^2$ un radical alkyle contenant de 1 à 4 atomes de carbone.

7. Procédé selon la revendication 1 caractérisé en ce qu'on effectue la réaction à une température comprise entre a et 120°C.

8. Procédé pour durcir des résines époxydiques, procédé caractérisé en ce qu'on fait réagir les composés de farmule II ou III selan la revendication 1 et en ce qu'on mélange les isomélamines de formule I ainsi obtenues avec des résines époxydiques.